# EUROPEAN PATENT APPLICATION

(11) **EP 0 535 337 A2**
(43) Date of publication of application: **07.04.1993**
(21) Application number: 92113632.1
(22) Date of filing: 10.08.1992
(51) Int. Cl.: A61K 37/36, C07K 13/00, G01N 33/68

(54) **Cell growth inhibiting activities of heparin binding neurite-outgrowth promoting factor**

(30) Priority: 30.09.1991 US 769063
(71) Applicant: AMERICAN CYANAMID COMPANY, Wayne, NJ 07470-8426 (US)
(72) Inventor: Backer, Joseph Mark, Tenafly, New Jersey 07670 (US); Bohlen, Peter, Peekskill, New York 10566 (US)
(74) Representative: Wächtershäuser, Günter, Prof. Dr.

(57) **Abstract**

The present invention relates to methods for inhibiting cell growth of such cells as endothelial cells by utilizing heparin binding neurite outgrowth prompting (HBNF).

## Description

The present invention relates to a method to inhibit cellular growth by the use of heparin-binding neurite-outgrowth promoting factor (HBNF) also referred to as heparin-binding neurotrophic factors, also previously referred to as heparin-binding brain mitogens (HBBMs).

The gene encoding HBNF has been isolated from a cDNA library and is a 411 nucleotide sequence having 136 amino acids in a protein with a molecular weight of about 15KD. The gene has been sequenced and expressed in E. coli, and the protein so produced retains the neurite-promoting activity of native HBNF.

Unexpectedly, the HBNF protein has been discovered to be an inhibitor of endothelial responses to basic fibroblast growth factor (bFGF) thereby indicating a potential inhibitory in vivo effect on growth.

### BACKGROUND OF THE INVENTION

In recent years, a number of relatively small polypeptides, known as growth factors, have been identified and isolated. The term "growth factors" refers to a class of signalling substances which affect the growth and differentiation of certain types of animals. This effect can be seen both in the animal and in tissue culture, and a given growth factor may have an effect on more than one type of cell.

Many of the better known growth factors have significant neurotrophic activity, i.e., they are capable of maintaining or stimulating growth of nerve cells. The earliest discovery of such a neurotrophic factor was nerve growth factor (NFG) (Levi-Montalcini and Hamburger, 1953). Similar growth factors which are in the same family as NFG are brain-derived neurotrophic factor (BDNF); (Leibrock et al., 1989) and neurotrophic factor - 3 (NT-3) (Maisonpierre et al., 1990). Additional growth factors include ciliary neurotrophic factor (CNTF) (Lin et al., 1990), IGF-II (Mill et al., 1985), activin (Schubert et al., 1990) purpurin (Berman et al., 1987) and also FGF (Baird and Bohlen, 1990).

A number of known growth factors fall into a superfamily related to fibroblast growth factor (FGF). This family includes basic FGF (bFGF) (Bohlen et al., 1984; Esch et al., 1985), acidic FGF (aFGF) (Bohlen et al., 1985; Gimenez-Gallego et al., 1985), as well as products of the oncogenes int - 2 (Dickens and Peters, 1984), hst/KS (Delli Bovi et al., 1987), FGF-5 (Zhan et al., 1988), FGF-6 (Marics et al., 1989) and KGF (Finch et al., 1989). These are all (except KGF) mitogens for vascular endothelial cells, and all also bind strongly to heparin. Other heparin - binding growth factors, such as VEGF/VPF, are also known (Keck et al., 1989). These heparin-binding growth factors are also frequently isolated from brain tissue and may play a significant role in the growth and development of brain cells.

A previously unknown heparin binding protein was described in EP 326 075, and recombinant gene disclosed in U.S. patent application Serial Number 07/568,574. The protein is structurally unrelated to any of the aforementioned growth factors, although it appears to be structurally related to a protein the gene of which was previously referred to as MK (Kadomatsu et al., 1988) with a human form of the MK protein disclosed in U.S. patent application Serial No. 07/568,473. The homology between HBNF and MK genes and proteins is very high, and they are assumed to constitute a new family of proteins with neurite-promoting and thus potentially neurotrophic activity.

More recently, HBNF proteins have been isolated from both rat (Rauvala, 1989, Huber et al. 1990), and cow (Milner et al., 1989; Huber et al. 1990; Bohlen et al., 1991), and the amino terminal sequences have been determined. Similarly, the N-terminal amino acid sequences of the human and chicken proteins have been determined (EP 326 075; Huber et al., 1990) Moreover, the DNA sequence of the HBNF gene has been disclosed in U.S. patent application Serial Number 07/568,574. HBNF cDNA was also cloned from cDNA libraries from various tissues (Li et al., 1990) and from osteoblasts (Tezuka, K. et al., 1990).

The present invention relates to the unexpected discovery that HBNF inhibits cellular growth.

The proliferation of endothelial cells and subsequent formation of new blood vessels are obligatory steps in the development of several pathological processes including tumor growth, arthritis, and retinopathies (Folkman & Klagsbrun, 1987). Furthermore, angiogenesis or the formation of new capillaries, is critical in a variety of pathological states or diseases. For instance, the following are but a few of the angiogenesis-dependent diseases: angiofibroma, arteriovenous malformations, arthritis, arthero-sclerotic plaques, corneal graft neovascularization, delayed wound healing, diabetic retinopathy, granulations-burns, hemangioma, hemophilic joints, hypertrophy scars, neovascular glaucoma, nonunion fractures, Osler-Weber Syndrome, psoriasis, pyogenic granuloma, retrolental fibroplasia, scleroderma, solid tumors, trachoma and vascular adhesions (Moses, et al., 1991). Basic fibroblast growth factor (bFGF) is a potent mitogen for endothelial cells in cell culture (Gospodarowicz et. al., 1984) and a powerful angiogenic factor in vivo (Baird & Bohlen, 1990). Endothelial cells express bFGF (Vlodavsky et al., 1987, Moscatelli et al., 1986, Hannan et al., 1987) and can use it as an autocrine growth factor (Sato & Rifkin, 1988). Studies of in vivo and in vitro localization of bFGF indicate that it is also associated with heparin or heparin sulfate moieties in the extracellular matrix and may be liberated by cellular enzymes to activate endothelial cells (Wanaka et al., 1991,Vlodavsky et al., 1987, Moscatelli, 1988, Bashkin et al., 1989, and Folkman et al., 1988). Therefore, developing specific and generally non-toxic antagonists of bFGF may be an effective approach to the therapeutic control of angiogenesis.

This strategy to control angiogenesis is particularly effective in view of recent progress in determining the mechanism of interaction between bFGF and cellular receptors. Most cells contain high affinity transmembrane glycoprotein receptors which bind bFGF with a K_{d}=10-¹⁰ to 10-¹¹M (Moscatelli, 1987). This type of receptor has been identified and several forms of it recently cloned from a variety of species (Ruta et al., 1988, Coughlin et al., 1988 Kornbluth et al., 1988, Lee et al., 1989, Pasquale & Singer, 1989, and Safran et al., 1990) thereby allowing more direct study of the interaction between growth affecting substances and receptors.

In addition to high affinity receptors, cells contain more numerous lower affinity receptors which are heparan sulfate proteoglycans and which bind bFGF with a K_{d}=10⁻⁹M (Moscatelli, 1987). The first member of this type of receptor has been cloned recently (Kiefer et al., 1990). A novel interaction between bFGF binding to low and high affinity receptors was recently reported by Yayon et al. (Yayon et al., 1991). This group showed that proper biosynthesis of cell surface heparan sulfate moieties is necessary for the binding of bFGF to the high affinity receptors. It was also shown that exogenous heparan could restore high affinity binding in cells expressing bFGF receptor but deficient in biosynthesis of heparin sulfates. Yayon et al. suggested that low affinity heparan sulfates proteoglycan receptors bind bFGF, induce conformational change in bFGF, and thus, "create" bFGF capable to bind to high affinity receptors. If the biosynthesis of heparan sulfate proteoglycans is impaired, exogenous heparin or heparan sulfate may induce the proper conformational change in bFGF. This "induced fit" model emphasizes the role of heparan sulfate proteoglycans in the presentation of bFGF to the receptor. In addition it suggests that occupancy of heparan sulfate proteoglycan receptors by heparin-binding proteins may inhibit bFGF binding to high affinity receptor. Indeed, it has been long known chat certain heparin-binding proteins are capable of inhibiting angiogenesis and endothelial cell growth in vitro (Taylor & Folkman, 1982, Dauchel et al., 1989). However, the mechanism of action is unknown.

More recently, experimental support for this model was provided by the finding that heparin-binding platelet derived protein PF4 (platelet factor 4) which displays angiostatic activity (Taylor & Folkman, 1982) also inhibits the binding of bFGF to high affinity receptors in NIH 3T3 cells (Sato et al., 1990). Additionally, while PF4 also inhibits both bFGF-driven migration of bovine and human endothelial cells (Sato et al., Sharpe et al., 1990) and bFGF-driven growth of human endothelial cells, it does not affect growth of other normal and tumor cells (Maione et al., 1990, 199]). However, Maione et al., recently presented new evidence which indicates that the mechanism of action of PF4 may not be directly associated with its ability to bind to heparin (Maione et al., 1990, 1991). This group found, that recombinant mutant PF4 and a synthetic C-terminal thirteen-mer fragment of PF4 which both lack affinity to heparin, still retain potent angiostatic activity in vivo, and mutant PF4 inhibits proliferation of human endothelial cells in vitro (Maione et al., 1990, 1991).

The heparin-binding protein HBNF is structurally unrelated to FGF and PF4 families of growth factors. But it has been discovered that native and recombinant HBNF proteins displace bFGF from high affinity binding sites on baby hamster kidney (BHK) cells and specifically inhibit growth of bovine and human endothelial cells in vitro, thereby indicating that the HBNF proteins are cellular growth inhibitors.

### BRIEF DESCRIPTION OF DRAWINGS

Figure 1: Effects of HBNF and carboxymethylated HBNF on bFGF binding to BHK cell high affinity receptors. ●-HBNF, ■-carboxymethylated HBNF. Assay is described in Example 5.

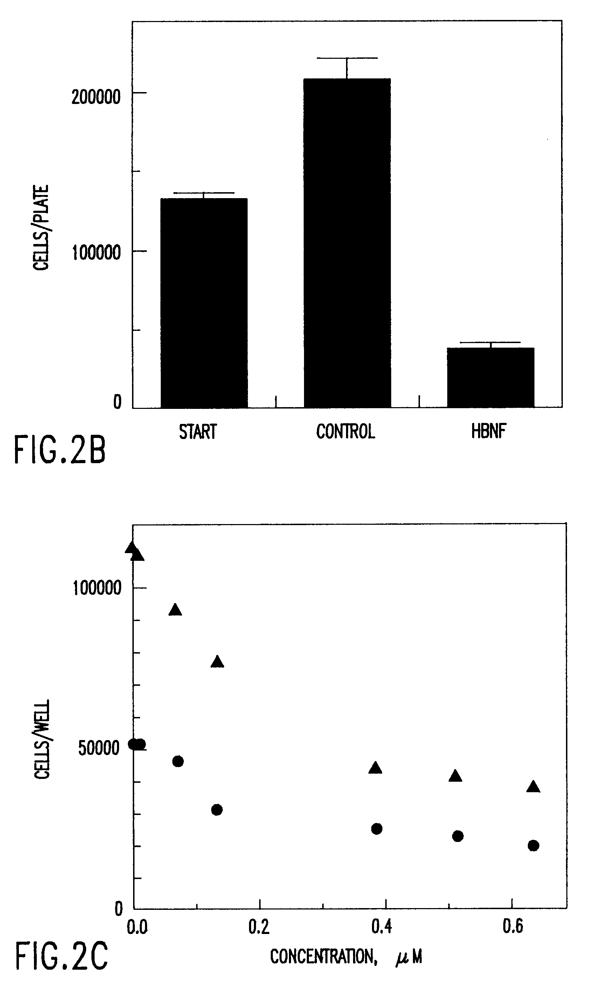

Figure 2: Effects of HBNF and carboxymethylated HBNF on bovine aortic endothelial (ABAE) cell growth. Assay is described in Example 6. Panel A: HBNF inhibits basal (●) and bFGF-stimulated (1 ng/ml) (▲) ABAE cell growth with ED₅₀=0.04µM. Panel B: 0.33µM HBNF causes detachment of ABAE cells. Subconfluent ABAE cells are exposed to 0.33µM HBNF for 4 days, during which a visible detachment of cells occurs. "Start"-refers to plates prior to addition of HBNF; "Control"-refers to plates which are not treated with HBNF; "HBNF"-refers to plates treated with HBNF. Assays are in duplicates. Panel C: carboxymethylated HBNF inhibits basal (●) and bFGF-stimulated (1 ng/ml) (▲) ABAE cell growth with ED₅₀=0.24µM and 0.13µM respectively.
Figure 3: Effect of HBNF on human umbilical cord endothelial (HUVE) cell growth. ●-1ng/ml bFGF in media; ▲-10 ng/ml bFGF im media. Assay is described in Example 6.
Figure 4: Effect of 0.65µM HBNF on human forskin fibroblast growth. Open bars -no bFGF, closed bars - 10 ng/ml bFGF in media. "Control" -refers to untreated cells; "HBNF"-refers to cells treated with 0.65µM HBNF for 4 days. Assay is described in Example 6.

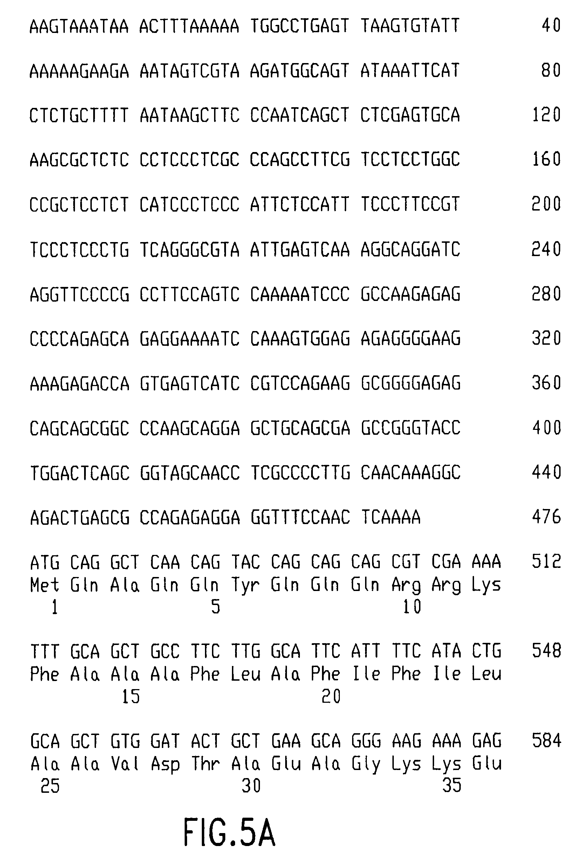

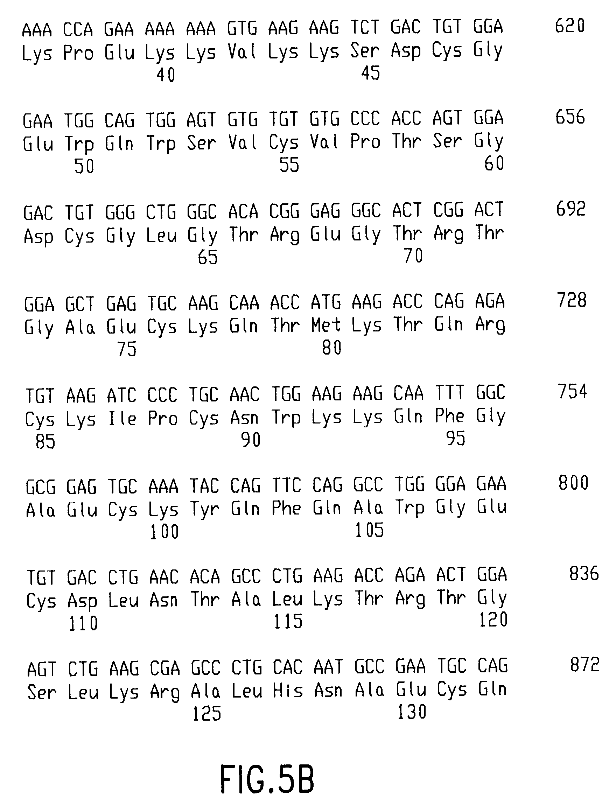

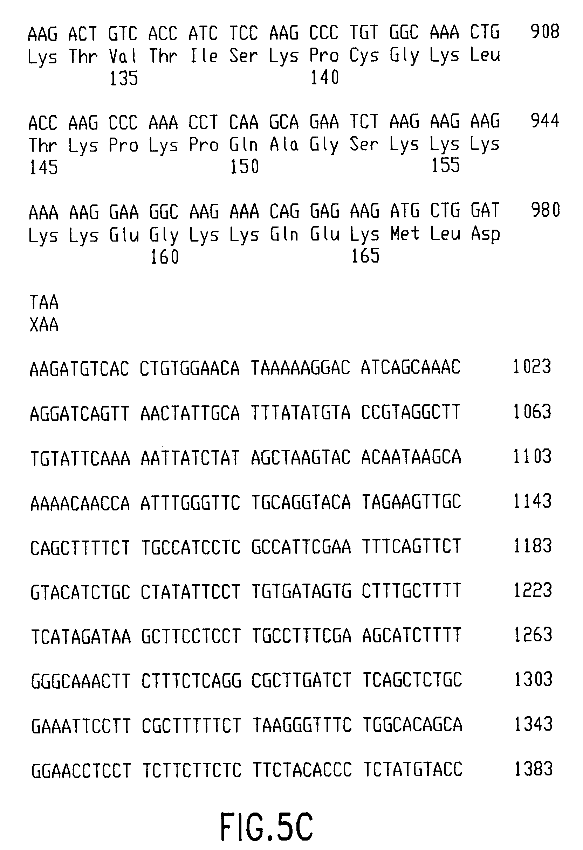

Figure 5: The complete sequence of HBNF is provided.

### SUMMARY OF THE INVENTION

The present invention relates to a method to inhibit cellular growth by administering HBNF to animals, especially warm-blooded animals. One type of cellular inhibition of tremendous consequence is inhibition of angiogenesis, the vascularization or formation of new blood vessels. Inhibiting vascularization in diseases such as solid tumors, rheumatoid arthritis and eye diseases such as retinopathies, neovascular glaucoma, ocular tumors and the like is important in the medical treatment of not only human beings, but may be useful in veterinary therapies, as well. In addition to treating disease states, the control of post-surgical bleeding in by-pass surgery, for instance, is also provided with the compounds of the present invention. Additionally, the use of the present invention can drive cells into a more differentiated state, thereby providing a chemotherapeutic use, as well. This may be achieved by direct administration of the purified protein or may also be achieved by transplant of transgenic host cells capable of producing the protein into the region of the body needing such treatment. Both natural sources and recombinantly-derived HBNF are contemplated for use in the present invention. Further, analogues of HBNF are useful in the present invention, as seen by the carboxymethylated forms disclosed for use herein as examples of alkylated HBNFs. Alkylation of one or more amino acid location on HBNF may provide more stable formulated forms of the protein for delivery in therapeutic use.

In addition to the above-discussed in vivo therapies, the compounds of the invention are useful in providing an in vitro screening mechanism to screen for FGF agonists and antagonists in responsive assays such as endothelial assays.

As such, the objectives of the present invention include providing methods for treating animals, warm-blooded in particularly, for angiogenesis-related diseases. Furthermore, the present invention also relates to methods for inhibiting cellular growth in animals, again, particularly in warm-blooded animals. This includes controlling tumor growth for potential chemotheropeutic regiments. Also, another objective relates to controlling post-surgical bleeding. The HBNFs useful in the present invention include but are not limited to human HBNF, bovine HBNF, ovine HBNF, porcine HBNF, caprine HBNF, feline HBNF, canine HBNF, avian HBNFs and fish NBNF. These and other objects of the invention will become apparent by the more detailed description of the invention provided hereinbelow.

The following examples illustrate the cloning and expression of the HBNF gene in a T7 RNA polymerase expression system. However, although this T7 expression system has proven quite efficient, it is to be understood that this is not the only means by which HBNF can be produced recombinantly. Production of HBNF can be achieved by incorporation of the HBNF gene into any suitable expression vector and subsequent transformation of an appropriate host cell with the vector. Alternatively, the transformation or the host cells can be achieved directly by naked DNA without the use of a vector. Production of HBNF by either eukaryotic cells or prokaryotic cells is contemplated as useful in the present invention. Examples of suitable eukaryotic cells include mammalian cells, plant cells, yeast cells and insect cells. Similarly, suitable prokaryotic hosts in addition to E. coli, include but are not limited to Bacillus subtilis.

Other suitable expression vectors may also be employed and are selected based upon the choice of host cell. For example, numerous vectors suitable for use in transforming bacterial cells are well known. For example, plasmids and bacteriophages, such as λ phage, are the most commonly used vectors for bacterial hosts, and for E. coli in particular. In both mammalian and insect cells, virus vectors are frequently used to obtain expression of exogenous DNA. In particular, mammalian cells are commonly transformed with SV40 or polyoma virus. Insect cells in culture may be transformed with baculovirus expression vectors. Yeast vector systems include yeast centromere plasmids, yeast episomal plasmids and yeast integrating plasmids.

It will also be understood that the method of the present invention is not limited to the exact sequence of the human HBNF gene and protein. Modifications to both the DNA and amino acids sequences, such as deletions, insertions, or substitutions in the DNA sequence which produce silent changes in the resulting protein molecule are also contemplated.

Furthermore, changes in the amino acids sequence which result in a biologically active HBNF analogue are also contemplated as useful in the present invention. For example, alterations in the gene sequence which reflect the degeneracy of the genetic code, or which result in the production of a chemically equivalent amino acid at a given site, are contemplated; thus, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a biologically equivalent product. Additionally, since it is primarily the central portion of the protein which is conserved among species, nucleotide changes which result in alteration of the N-terminal and C-terminal portions of the protein molecule, would not be expected to alter the activity of the protein. Indeed, the "HBBM" size variants disclosed in EP 326,075 include C-terminal truncations of the HBNF protein. It may also be desirable to change a hydrophobic amino acid to a charged amino acid if no adverse effect is observed on the biological activity. Each of the proposed modifications is well within the routine skill in the art, as is determination of retention of biological activity of the encoded products. Therefore, where the phrase "HBNF" is used in either the specification and the claims, it will be understood to encompass all such modifications and variations which result in the production of a biologically equivalent HBNF protein. In particular, the invention contemplates those proteins which are sufficiently duplicative as to permit hybridization therewith under standard high stringency Southern hybridization conditions, such as those described in Maniatis et al., (Molecular Cloning. A Laboratory Manual. Cold Spring Harbor Laboratory, 1982).

The identification of the gene and its sequence permit construction of transgenic cells such as fibroblasts, monocytes, or macrophages, which may be engineered to permit expression of the HBNF gene and used as an implant for treatment of the diseases previously discusses.

Moreover, the therapeutic use of HBNF is not limited to treatment of human beings alone. In fact, in view of the conserved nature of this protein among distantly related species, administration of HBNF in any form may be beneficial for veterinary application as well. Therapeutic compositions comprise HBNF in amounts sufficient to produce the desired biological effect, in combination with a pharmaceutically acceptable liquid or solid carrier. Alternately, the composition comprises a pharmaceutically acceptable aggregation of compatible transgenic cells capable of expressing HBNF in vivo, as an implant for peripheral and central nervous system repairs or differentiation treatment.

The following examples are presented for purposes of illustration only and are not to be considered as limiting the scope of the present invention.

### Example 1

### HBNF Protein Purification, Cloning and Expression and Amino Acid Sequence Analysis

HBNF protein is isolated from bovine brain by heparin-sepharose affinity chromatography and mono-S cationic exchange chromatography, described previously in EP 326 075, which is incorporated herein by reference in its entirety. Briefly, reverse-phase HPLC-purified HBNF is chemically modified by reduction in mercaptoethanol and alkylation of cysteine residue with iodo-(2-14C)-acetic acid according to a procedure described previously (Gautschi-Sova et al., 1986) Carboxymethylated protein is purified by reverse-phase HPLC using a Brownlee Aquapore C8 column (25 X 0.46 cm 7 um particle size, Applied Biosystems) using as the mobile phase 0.1% trifluoroacetic acid in an acetonitrile gradient. Aliquots corresponding to 3 nmol of carboxymethylated HBNF are diluted with enzyme digestion buffer to reduce the acetonitrile concentration of the sample to approximately 10% and digested with the following proteases: Staphylococcus aureus V8 (cleavage after glutamic acid residues), Arg-C (cleavage after arginine), Asp-N (cleavage before aspartic acid) and chymotrypsin (preferential cleavage after armatic residues). Enzymes are from Boehringer Mannheim and cleavage reactions are performed essentially as suggested by the manufacturer. After digestion, peptides are separated by reverse phase HPLC on a C8 column using a 90-min linear gradient of acetonitrile in 0.1% trifluoroacetic acid for peptide elution (acetonitrile content at start: 12-16%, at end: 30-44%, depending on the type of digest). In order to ascertain homogeneity of purified peptides, fractions containing peptide material are subjected to a second reverse-phase HPLC step (C8 column, 0.1% heptaflurobutyric acid in an appropriate shallow acetonitrile gradient). Aliquots of 5-500 pmol of isolated peptides are sequenced on an Applied Biosystems 477A gas/liquid-phase microsequenator. Phenyl thiohydantoin (PTH) amino acid derivatives are identified on a Model 120A on-line PTH amino acid analyzer (Applied Biosystesm). Experimental protocols for both procedures are as supplied by the instrument manufacturer.

The bovine HBNF amino acid sequence is used to design degenerate oligonucleotides for the PCR amplification reaction. A completely degenerate sense primer is made to the amino acid sequence: Asp-Cys-Gly-Glu-Trp-Gln-Trp starting with a HindIII restriction site and comprised of the DNA sequence:
5'-CAAGCTTGGAPyTGPyGGNGAPuTGGCAPuTGG-3'. A completely degenerate antisense primer is made to the amino acid sequence: Asn-Ala-Asp-Cys-Gln-Lys-Thr starting with an EcoRI restriction site and comprised of the DNA sequence:
5'-GGAATTCCGTPyTTPyTGPuCAPuTCNGCPuTT-3'.

Total rat brain RNA is isolated from the brains of Sprague-Dawley rats by the guanidinium isothiocyanate- cesium chloride method and poly (A) + RNA is selected by two cycles of binding to oligo (dT) - cellulose (Aviv and Leder, 1972). The rat brain poly (A) + RNA is reverse transcribed with oligo (dT) and AMV reverse transcriptase (Maniatis et al., 1982.) The PCR reaction is carried out on the complementary DNA template, with 30 cycles, with one minute annealing at 50^{o}C, two minutes extension at 72^{o} and one minute denaturation at 94^{o}C for 30 cycles using Taq polymerase (USB).

The 282 base pair rat HBNF PCR product is cloned into Blue Scribe (+) vector (Stratagene) and used as a probe in screening a newborn human brainstem and basal ganglia λ gt 11 cDNA library. Thirty HHC clones are initially identified and after preliminary restriction analysis, four clones are isolated, subcloned in the EcoRI site of Blue Scribe (+), and sequenced by the dideoxynucleotide chain termination method (Sanger et al., 1988).

Three of the clones have identical sequences in the coding region and the fourth clone has a three-nucleotide in-frame deletion resulting in the removal of an alanine at position 119.

Clone HHC8 is chosen for use as a template for PCR amplification with primers designed to place a methionine codon and an Ndel restriction site immediately 5' to the N-terminal glycine. The purified PCR product is cloned into a derivative of the expression vector pET-3a, which is modified by the deletion of the 1400 bp Sa11/PvuII fragment and insertion of an f1 origin of replication into the EcoRI site. After sequencing the insert to confirm the fidelity of the PCR amplification, the plasmid (named pETHH8) is transformed into strain BL21 lysS and induced for protein production with IPTG. Pellets from one ml cultures are resuspended in 100 µl of SDS buffer (Laemmli, 1970) and 2.5 ul run on a 15% acrylamide SDS-PAGE gel. The gel is stained with coomassie blue. Native HBNF is purified from rat brains and recombinant HBNF from bacterial extract on heparin sepharose CL-6B (Pharmacia) resin in 10 mM Tris, pH 7.0 and eluted with a gradient from 0-2 M NaCl at 1-1.13 M NaCl. Further purification is achieved on Mono S (Pharmacia) columns in 50 mM sodium phosphate, pH 6.8, using a gradient of increasing salt concentration from 0 to 1 M NaC1 for elution.

The expression of the HBNF gene is investigated in mouse tissues. Total cellular RNA is isolated by the guanidinium isothiocyanate-cesium chloride method, analyzed on 1% agarose gel containing 0.66 M formaldehyde and blotted onto nylon membrane filter formamide with ³²P labeled cDNA probes prepared by random oligonucleotide priming. The filters are washed at 65^{o}C in 1xSSC (0.15 M NaCl, 15 mM Na-citrate pH 7.0), 0.2% SDS and exposed to X-ray films. Northern hybridization analysis of mouse RNA from a variety of tissues using human HBNF cDNA as probe indicates that only the brain expressed a 1650 nucleotide message. Analysis of total human RNA indicates that the human mRNA is approximately 1600 nucleotides in length, slightly shorter than that of the mouse.

An E. coli, strain BL 21 lysS, harboring plasmid pETHH8, has been deposited in American Cyanamid Company's Culture Collection maintained in Pearl River, New York, and with the American Type Culture Collection, 12301 Parklawn Drive, Rockville, Maryland, on August 13, 1990, under accession number ATCC 68385 and is available to the public pursuant to the appropriate legal standards for patents in the U.S. and other countries.

### Example 2

### Other proteins

Human platelet-derived PF4 is purchased from Sigma in St. Louis. Recombined bFGF and analogues with wildtype activity and potency are expressed and purified as described in (Seddon et al., 1991). ¹²⁵I-bFGF (1,000Ci/mole) is purchased from Amersham.

### Example 3

### Carboxymethylated HBNF

Carboxymethylated HBNF is prepared as follows: Lyophilized recombinant HBNF is dissolved in 0.1M Tris-HC1 pH 8.6, containing 2mM EDTA and 4.5M guanidinium HC1 to give a concentration of 0.5 mg/ml. The protein is reduced with dithiothreitol (5mM) and the solution incubated under an argon atmosphere for 1 hour at 37^{o}C. The reduced protein solution is cooled to room temperature and alkylated using iodoacetic acid (15mM) for 1 hour in the dark. The carboxymethylated protein is dialysed (3500 molecular weight cut-off) overnight at 4^{o}C versus 10mM Tris-HC1 pH 7.2 containing 200mM NaC1. Carboxymethylcysteine and protein concentrations are determined by amino acid analysis after HC1 gas phase hydrolysis (5.7M HC1/01.%phenol;24h at 100^{o}C) using a model 420A PITC-derivatizer equipped with an on-line model 130A separation system (Applied Biosystems, CA). Carboxymethylated HBNF is eluted from Heparin-sepharose with 0.9M NaC1, while native HBNF is eluted with 1.1M NaCl.

### Example 4

### Cells Lines

BHK cells are grown in Dulbecco's Modified Eagle Medicine (DMEM) (Mediatech, Washington, D.C.) supplemented with 5% fetal calf serum and 5% calf serum (Gibco). Bovine ABAE cells are grown in DMEM supplemented with 10% calf serum (Hy Clone). Human foreskin fibroblasts (HFSF) and human melanoma cell lines, gifts from Dr. Eisinger (Lederle Laboratories, Pearl River, NY), are grown in MEM media (Mediatech, Washington, D.C.) supplemented with 7.5% fetal calf serum (Gibco). Human umbilical vein endothelial (HUVE) cells (passage 1), a gift from Dr. Jaffe (Cornell University, Medical Center, New York, NY) and are grown in Media 199 (Mediatech, Washington, D.C.) supplemented with 4% serum (Gibco) and 16% fetal calf horse serum (Gibco) as described by Jaffe, 1984.

### Example 5

### Radioreceptor Assay

¹²⁵I-bFGF binding to high affinity receptors on BHK cells is performed according to Moscatelli (1987). Briefly, cells are incubated with 50pM of ¹²⁵I-bFGF and various additions for 1 hour at room temperature, then incubated at 4^{o}C for 30 minutes. High salt (2M) washing of the cells releases bFGF from low affinity sites, while treatment of the cells with 0.5% Triton X100 releases bFGF from high affinity sites. The assay is validated by a displacement experiment in which increasing concentrations of non-radioactive bFGF are added to the incubation mixture. Non-radioactive bFGF decreases the amount of radioactivity released by Triton X100 in a dose-dependent manner with an ED₅₀ of 50pM. Binding assays are done in duplicates.

### Example 6

### Mitogenic Assays

Mitogenic assays are performed according to Fafeur et al. (1990). Briefly, bovine ABAE cells are plated in multi-well dishes at 8,000 cells/well with or without 1ng/ml bFGF for 4 hours, followed by addition of increasing concentrations of heparin-binding proteins. In the presence of 1ng/ml of bFGF the number of ABAE cells is 3-4 fold higher than in the absence of bFGF. HUVE cells are seeded in gelatin coated 24-well plates at 8,000 cells/well and compounds are added 16 hours after seeding. After four days, cells are detached and counted. Mitogenic assays are done in duplicates.

### Example 7

### Neurite Outgrowth Assays

Brains from 18-day fetal rats are removed under sterile conditions and dispersed to single cells in DMEM containing 10% FCS using a sterile 5 ml syringe. The cell suspension as adjusted to 5 x 10⁵ cells/ml and plated onto tissue culture dishes that are precoated with 50µg/ml poly-L-lysine for 30 minutes at room temperature (Rauvala and Pihlaskari, 1987). Cultures are incubated for 24 hours at 37^{o}C in 10% Co₂, after which the media are changed to DMEM containing 1 mg/ml BSA, and HBNF or MK proteins are added at indicated concentrations. After a further one-day incubation, neurite outgrowth activity is determined by visual examination of cells for extended outgrowth/processes as compared to controls.

### Example 8

### Growth and Retinoic Acid Induction of the Human NT2/D1 Cells

The human embryonal carcinoma cell line Nt2/D1 is grown as described previously. For retinoic acid induction, cells are grown and resuspended in DMEM medium containing 10% bovine calf serum and resuspended in DMEM medium containing 10% bovine calf serum (Hyclone Laboratories, Inc.) at a density of 5 x 10⁵ cells per 100 mm dish. Varying concentrations of all-trans retinoic acid in dimethyl sulfoxide (10 µl) is added and cells are incubated for 9 days. Fresh medium and RA are added at days 4 and 8. Plates are washed once with phosphate buffered saline, and RNA extracted as described above. Figure 8 shows a graphic representation of the levels of both HBNF and MK produced in response to varying levels of retinoic acid concentration. Since NT2/D1 cells induced with RA have been suggested as providing a model system for studies of neuronal differentiation, the increase in induction of HBNF and MK genes in this system indicates a possible role in neuronal cell development.

### Example 9

### Results of Assays and Binding Studies

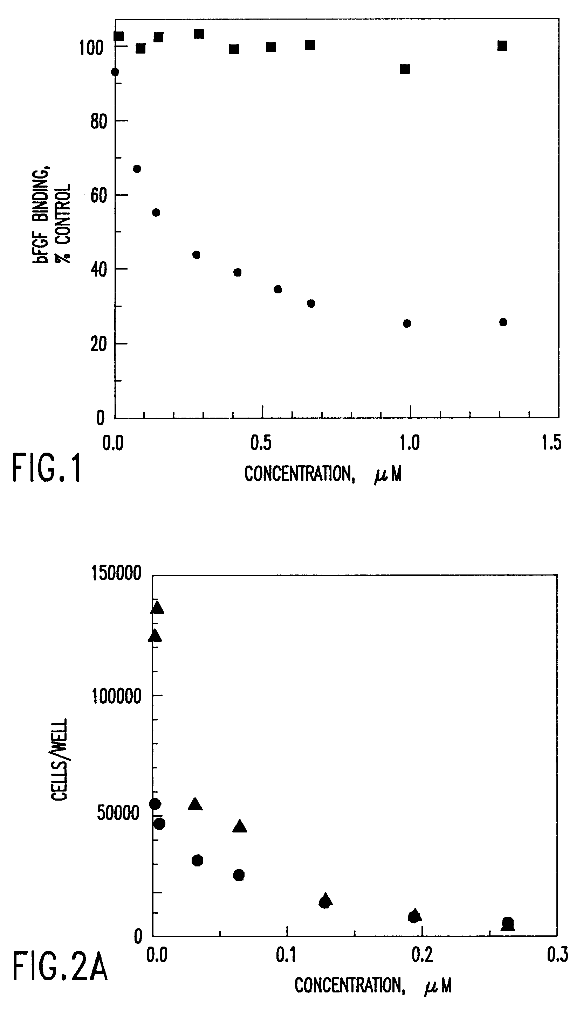

The effects of HBNF on bFGF binding to high affinity receptors of BHK cells disclose that HBNF inhibits bFGF binding with an ED₅₀ of 0.1µM (Figure 1). The inhibitory activity of HBNF is destroyed by carboxymethylation of the protein's ten cysteine residues (Figure 1). This treatment presumably prevents proper folding of HBNF and decreases the HBNF affinity for Heparin-Sepharose.

Binding of bFGF to high affinity sites on BHK cells is also inhibited in the presence of human platelet factor pF4, another heparin-binding protein. Like HBNF, PF4 inhibits bFGF binding in a douse-dependent manner with an ED₅₀ of 0.25µM. The latter value is identical to that deduced from data obtained in similar experiments with NIH 3T3 (Sato et al., 1990).

Next, the effects of HBNF on basal and bFGF-stimulated growth of bovine endothelial ABAE cells are assayed. HBNF inhibits basal and bFGF-stimulated growth of ABAE cells in a dose-dependent manner with a similar ED₅₀ of 0.04µM (Figure 2A). High concentrations of HBNF (above 0.2 µM) not only completely inhibit growth of ABAE cells but also cause visible detachment of ABAE cells from the plate (Figure 2, panels A and B).

Carboxymethylated HBNF inhibits growth of ABAE cells (Figure 2C) even in light of the fact that it seems not to displace bFGF from BHK cell receptors (see Figure 1). The ED₅₀ for carboxymethylated HBNF is 2-3 fold higher than that for HBNF.

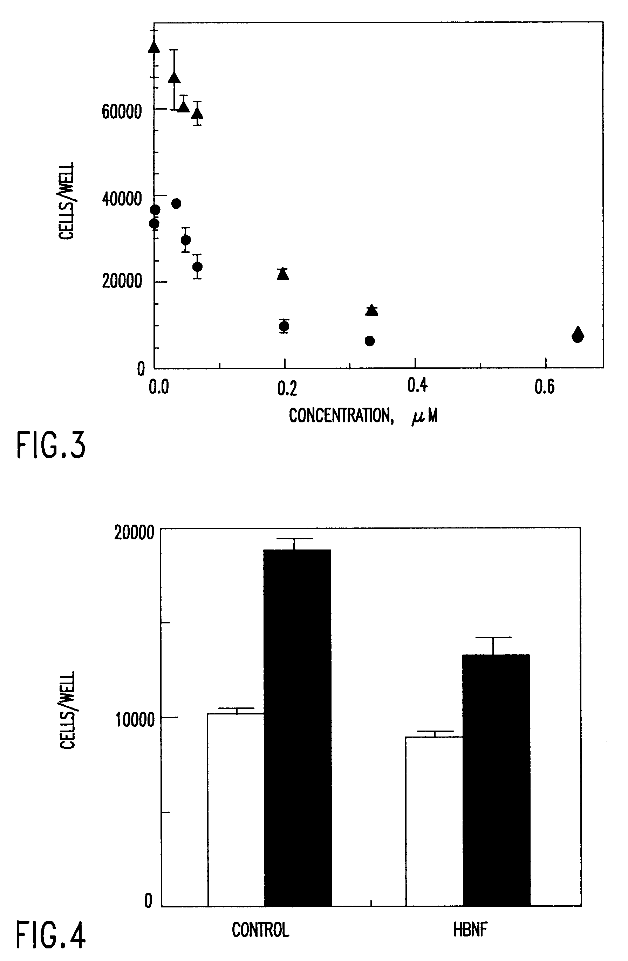

HBNF also inhibits bFGF-stimulated growth of human primary endothelial cells (HUVE cells) at bFGF concentrations of 1 ng/ml and 10 ng/ml (Figure 3). The bFGF-stimulated growth of HUVE cells is inhibited by HBNF in a dose-dependent manner at both concentrations of bFGF with similar ED₅₀ values. However, high concentrations of HBNF (above 0.2 µM) inhibit HUVE cell growth to a lesser extent at 10 ng/ml bFGF than at 1 ng/ml bFGF (Figure 3). Unlike bovine ABAE cells, human HUVE cells are not inhibited by carboxymethylated HBNF (Figure 3).

An anti-proliferatory activity of HBNF is not observed with hamster BHK cells and rat PC12 cells. These proteins also do not affect growth of two non-endothelial human cell lines: normal human foreskin fibroblasts (HFSF) and a highly malignant melanoma cell line. With both cell lines 0.65 µM (10µg/ml) of HBNF does not affect bFGF independent cell growth (data for human foreskin fibroblasts are shown on Figure 4). The presence of 10 ng/ml bFGF in the media results in small but significant increases in the numbers of fibroblasts (but not melanoma cells). This bFGF stimulated growth is not observed in the presence of 0.65µM HBNF (Figure 4).

While not wanting to be limited by theory, the following is provided as a possible discussion of the above results.

HBNF, bFGF, and PF4 belong to different protein families and thus direct competition for binding to the high affinity bFGF receptor seems unlikely. However, HBNF, as well as PF4, may compete with bFGF for binding to the heparan sulfate moieties of low affinity bFGF receptors. As discussed hereinabove, recent findings indicate that binding of bFGF to low affinity receptors is necessary for creation of an "induced fit" conformation of bFGF able to bind to high affinity receptors (Yayon et al., 1991). Functional studies also indicate that intact low affinity receptors are necessary for bFGF-stimulated fibroblast growth and myoblast differentiation (Rapraeger et al., 1991). Thus, according to the above proposed model, the occupation of low affinity receptors by HBNF and PF4 is expected to decrease the number of bGFG molecules capable of binding to high affinity receptors.

The affinity of HBNF for cellular heparan sulfate proteoglycans is not known. Affinity of human PF4 for heparan sulfate proteoglycans of bovine endothelial cells is recently characterized (Kd=2.87µM, Rybak et al., 1989). If HBNF and PF4 have similar affinities for heparan sulfate, then one might expect that micromolar of submicromolar concentrations of these proteins saturate low affinity receptors for bFGF. It is noteworthy, that, as judged by ED₅₀ recombinant HBNF is 2.5 fold more potent then human platelet-derived PF4 in inhibiting bFGF binding to high affinity BHK cell receptors.

Moreover, a comparison of the ED₅₀ obtained in mitogenic assays on human endothelial cells with recombinant PF4 (Maione et al., 1990, 1991) and recombinant HBNF demonstrates that the latter protein is 5-10 fold more potent. This difference may be important in view of cytotoxicity of some heparin-binding proteins at high concentrations.

Results obtained with bovine ABAE cells indicate that high concentrations of HBNF (above 0.2 µM) cause the detachment of cells. It is not clear whether this detachment is directly caused by HBNF or is a consequence of HBNF cytotoxicity. Experiments with human HUVE cells demonstrate that the effect of high concentrations of HBNF may be counteracted by raising the concentration of bFGF. It appears that HBNF affects only endothelial cells, but not human fibroblasts, human melanoma cells, rat PC12 cells and hamster BHK cells.

In view of these findings it is suggested that additional "cell-mediated" mechanisms of inhibition are involved in the anti-proliferatory activity of heparin-binding proteins directed toward endothelial cells since inhibitory effects of HBNF have similar values at different concentrations of bFGF suggesting that direct competition for the receptor is not the sole factor.

Similarly, Maione et al., (1991) found that although a recombinant mutant of PF4 lacked affinity to heparin, it still retained potent angiostatic activity in vivo and inhibited proliferation of human endothelial cells in vitro thereby also suggesting various "cell-mediated" mechanisms of inhibition.

### Bibliography

1. Baird, A., and Bohlen, P. (1990) Fibroblast growth factors. In: Peptide growth factors and their receptors 1. (eds. Sporn, M.B., and Roberts, A.B.) pp369-418. Spring-Verlag, Berlin, Heidelberg.
2. Moscatelli, D., (1987). J. Cell Physiol 131:123-130.
3. Ruta, M., Howk, R., Ricca, G., Drohan, W., Zabelshansky, M., Laureys, G., Barton, D.E., Francke, U., Schlessinger, J., and Givol, D., (1988). Oncogene 3:9-15.
4. Coughlin, S.R., Barr, P.G., Cousens, L.S., Fretto, L.J., and Williams, P.T., (1988) kinase activity in vivo. J. Biol. Chem. 263:928-933.
5. Kornbluth, S., Paulson, K.S., and Hanafusa, H. (1988) Mol. Cell Biol. 8:5541-5544.
6. Lee, P.L., Johnson, D.E., Cousens, L.S., Fried, V.A., and Williams, L.T. (1989) Science 245: 57-60).
7. Pasquale, E.B., and Singer, S.J. (1989) Proc. Natl. Acad. Sci. USA 86:5449-5453.
8. Safran, A., Avivi, A., Orr-Urtereger, A., Neufeld, G., Lonai, P., Givol, D., and Yarden, Y. (1990) Oncogene 5:635-643.
9. Kiefer, M.C., Stephans, J.C., Crawford, K, Okino, K., and Barr, P.J. (1990) Proc. Natl. Acad. Sci. USA 87:6985-6989.
10. Vlodavsky, I., Folkman, J., Sullivan R., Freidman, R., Ishai-Michaeli, R. Sasse, and Klugsbrun, M. (1987) Proc. Natl. Acad. Sci. USA 84:2292-2296.
11. Moscatelli, D. (1988) J.Cell Biol. 107:753-759.
12. Bashkin, P., Doctrow, S., Svahn, C.M., Folkman, J., and Vlodavsky, I. (1989) Biochemistry 28,1737-1743.
13. Folkman, J., Klagsbrun,M.,Sasse,J., Wadzinski,M., Ingber,D., and Vlodavsky, I. (1988) J. Pathol. 130:393-400.
14. Yayon, A., Klagsbrun, M., Esko, J.D., Leder, P., and Ornitz, D.M. (1991) Cell, 64:841-848.
15. Taylor, S., and Folkman, L. (1982) Nature 297:307-312.
16. Sato, Y., Abe, and Takaki, R. (1990) Biochem. Biophys.Res. Commun. 172:595-600.
17. Sharpe, R.J., Byers, H.R., Scott, C.F., Bauer, S.I., and Maione, T.E. (1990) J.Natl. Cancer Inst. 82:848-853.
18. Maione, T.E., Gray, G.S., Petro, J. Hunt, A.T., Donner, A.L., Bauer, S.I., Carson, H.F., and Sharpe, R.G. (1990) Science (Washington, DC) 247:77-79.
19. Maione, T.E., Gray, G.S., Hunt, A.J., and Sharpe, R.J. (1991) Cancer Res. 51:2077-2083.
20. Kretschmer, P.J., Fairhurst, J.L., Decker, M.M., Chan, C.P. Gluzman, Y., Bohlen, P., and Kovesdi, I. (1991) Growth Factors 5:99-114.
21. Fafeur, V., Terman, B.I., Blum, J., and Bohlen, P. (1990) Growth Factors 3:237-245.
22. Jaffe, E.A. (1984) Culture and identification of large vessel endothelial cells. in Biology of Endothelial Cells. (ed. Jaffe, J.A.), Martinus-Nijhoff, New York.
23. Gospodarowicz. A., Cheng, J., Lui, G.M., D., Baird, and P. Bohlen (1984) Prox. Natl. Acad. Sci. USA 81:6963-6967.
24. Moscatelli, D.,Presta,M., Joseph-Silverstein, J., and Rifkin, D.B. (1986). J. Cell Physiol. 129: 273-276.
25. Hannan, R.L., Kourembanas, S., Flanders, K.C. Rogelj, S.J., Roberts, A.B., Faller, D.V., and Klagsbrun, M. (1987) Growth Factors 1:7-17.
26. Sato, Y., and Rifkin, D.B. (1988) J. Cell Biol. 107:1199-1205.
27. Maione, T.E., and Sharpe, R.J. (1990) TIBS 11:457-461.
28. Folkman, J., and Klagsbrun, M., (1987) Science (Washington DC) 235:442-447.
29. Seddon, A., Decker, M., Muller, T., Armellino, D., Kovesdi, I. Gluzman, Y., and Bohlen,P., (1991). Annals New York Acad. Sci., (in press).
30. Dauchel, M.C., Courty, J., Mereaii, A., and Barritault, D., (1989). J. Cell. Physiol. 39:411-420.
31. Rapraeger. A.C., Krufka, A., and Olwin,B.B. (1991) Science (Washington, DC) 252:1705-1708.
32. Rybak, M.E., Gimrone, Jr., M.A., Davies, P.F., and Handin, R.I., (1989) Blood, 73:1534-1539.
33. Wanaka, A., Milbrandt, J., and Johnson, E.M., (1991) Development 111,455-468.
34. Gospodarowicz, D. (1975) Biol. Chem 250:2515-2520, 1975.
35. Leibrock et al. (1989) Nature; 341:149-153
36. Maison pierre et al. (1990) Science 247:1446-1451.
37. Lin et al. (1990) Science 246:1023-1025.
38. Mill et al. (1985) PNAS USA 82:7126-7130.
39. Schubert et al. (1990) Nature 344:868-870.
40. Berman et al. (1987) Cell 51:135-142.
41. Bohlen et al. (1984) PNAS USA 81:5364-5368.
42. Esch et al. (1985) PNAS USA 82:6507-6511).
43. Bohlen et al. (1985) Embo J. 4:1951-1956.
44. Gimenez - Gallego et al. (1985) Science 230:1385-1288.
45. Dickens and Peters (1984) Nature 326:833.
46. Delli Bovi et al. (1987) Cell 50:729-737.
47. Zhan et al. (1988) Cell Biol 8:3487-3495.
48. Marics et al. (1989) Oncogene 4:335-340.
49. Finch et al. (1989) Science 245:752-755.
50. Kadomatsu et al. Biochem (1988) Biophys Res Comm 151:1312-1318.
51. Rauvala, H. (1989) EMBOJ 8:2933-2941.
52. Huber et al. (1990) Neurochem. Res. 15:435-439.
53. Gautschi-Sova et al. (1986) Biochem. Biophys. Res. Comm. 140:1874-1880.
54. Aviv and Leder, (1972) PNAS USA 69:14088-14120).
55. Maniatis et al. (1982) Molecular Cloning. A Laboratory Manual, Cold Spring Harbor Laboratory, Cold Spring Harbor, NY).
56. Sanger et al. (1988) PNAS USA 74:5463-5467.
57. Milner et al. (1989) Biochem Biophys. Res. Comm. 165:1096-1103.
58. Moses et al. (1991) Biotechnology, 9:630-634.
59. Levi-Moutalcini R. and Hamburger V. (1953) J. Exp. Zool. 123:233-278.
60. Bohlen et al. (1991) Growth Factors 4:97-108.
61. Li et al. (1990) science 250:1690-1694.

## Claims

1. The use of herparin binding neurite-outgrowth promoting factor (HBNF), alkylated HBNF, or a combination thereof for the manufacture of a composition useful for inhibiting cell growth.

2. The use of Claim 1, wherein the HBNF or alkylated HBNF is human HBNF, bovine HBNF, ovine HBNF, canine HBNF, porcine HBNF, feline HBNF, equine HBNF, avian HBNF, fish HBNF or the alkylated form thereof.

3. The use of HBNF, alkylated HBNF, or a combination thereof for the manufacture of a composition useful for controlling angiogenesis in an animal.

4. The use of Claim 3, wherein the HBNF or alkylated HBNF is human HBNF, bovine HBNF, ovine HBNF, canine HBNF, porcine HBNF, feline HBNF, equine HBNF, avian HBNF, fish HBNF or the alkylated form thereof.

5. The use of HBNF, alkylated HBNF, or a combination thereof, for the manufacture of a composition useful for controlling post-surgical bleeding in a warm-blooded animal.

6. The use of Claim 5, wherein the HBNF or alkylated HBNF is human HBNF, bovine HBNF, ovine HBNF, canine HBNF, porcine HBNF, feline HBNF, equine HBNF, avian HBNF, fish HBNF or the alkylated form thereof.

7. The use of HBNF, alkylated HBNF, or a combination thereof for the manufacture of a composition useful for inhibiting tumor growth in an animal.

8. The use of Claim 7, wherein the HBNF or alkylated HBNF is human HBNF, bovine HBNF, ovine HBNF, canine HBNF, porcine HBNF, feline HBNF, equine HBNF, avian HBNF, fish HBNF or the alkylated form thereof.

9. A method for screening in vitro for a cellular growth inhibitor, the method comprising: administering HBNF in a responsive cell assay.
